# EUROPEAN PATENT APPLICATION

(11) **EP 1 930 499 A1**
(43) Date of publication of application: **11.06.2008**
(21) Application number: 06025407.5
(22) Date of filing: 08.12.2006
(51) Int. Cl.: D21H 21/22, A61K 8/02, A61K 8/06, D21H 27/00

(54) **Tissue paper comprising a softening lotion**

(71) Applicant: Cognis IP Management GmbH, 40589 Düsseldorf (DE)
(72) Inventor: Urban, Andrea, 67069 Ludwigshafen (DE); Eichhorn, Dr. Stephan, 64579 Gernsheim (DE); Sorns, Jörg, 40476 Düsseldorf (DE); Kawa, Rolf, 40789 Monheim (DE)

(57) **Abstract**

The present invention relates to a tissue paper comprising a lotion composition, said lotion being a liquid W/O emulsion comprising
(A) a hydrocarbon-based oil,
(B) optionally at least one further oil,
(C) 3 to 40 weight % of at least one non-ionic W/O emulsifier,
(D) 0.5 to 10 weight % of at least one coemulsifier based on an ethylene oxide-modified glyceride,
(E) optionally 1 to 15 weight % of at least one humectant,
(F) optionally up to 10 weight % of a metal soap
(G) 6 to 35 weight % of water,

wherein the weight % values relate to the total weight of the lotion composition.

The lotioned tissue paper of the invention is very stable, shows excellent softness properties and is capable of efficiently transferring lotion to the skin of the user. Moreover, the lotion can be applied at relatively low temperatures and does not adversely affect the whiteness degree of the tissue paper.

## Description

### Field of invention

The invention relates to a soft lotioned tissue paper, in particular, to a soft lotioned tissue paper having softness due to a specific lotion composition which penetrates tissue paper.

### Background art

Softness is an important property of tissue products such as handkerchiefs, cosmetic wipes, toilet paper, serviettes/napkins, not to mention hand or kitchen towels, and it describes a characteristic tactile sensation caused by the tissue product upon contact with the skin.

Although the term "softness" is generally comprehensible, it is extremely difficult to define because there is no physical method of determination and, consequently no recognized industrial standard for the classification of different degrees of softness.

To be able to detect softness at least semi-quantitatively, softness is determined in practice by means of a subjective method. To do so, a "panel test" is carried out in which several trained test persons give a comparative opinion.

In simplified terms, softness can be subdivided into its main characteristics, surface softness and bulk softness.
Surface softness describes the feeling perceived when e.g. one's fingertips move lightly over the surface of the sheet of tissue. Bulk softness is defined as the sensory impression of the resistance to mechanical deformation that is produced by a tissue or tissue product manually deformed by crumpling or folding and/or by compression during the process of deformation.

One method for increasing bulk softness of tissue paper as taught by WO 96/25557 involves
a) wet-laying an aqueous slurry containing cellulosic fibres to form a web
b) applying a water soluble polyhydroxy compound to the wet web, and
c) drying and creping the web (wet web addition method).

It is further known from US4,764,418 that some humectants such as polyethylene glycol contribute to the softness of tissue products if they are applied to a dry web.

The use of humectants, such as polyhydroxy compounds, in highly concentrated form, as softeners however, has the disadvantage that the humectant may, upon contact, draw too much moisture from the skin, for instance when blowing one's nose with a tissue handkerchief. Moreover the softening effect is not yet satisfactory.

WO 96/24723 teaches increasing the surface softness of tissue paper by applying discrete deposits of a water free lotion composition containing an oil and a wax. Since however, due to its solid consistency, the treatment composition remains on the surface of the tissue paper, it cannot contribute to bulk softness. Further, water-free lotion compositions based on waxy or oily materials often feel unpleasantly greasy or oily.

Moreover, water-free lotions such as the one in WO96/24723 often do not feel particularly pleasant to the skin which is due to their low moisture content. This holds true also for the water-free lotions of WO 95/35411, WO 95/35412 and WO 95/16824 which are used for softening tissue paper, too.

WO 97/30216 discloses liquid, high water softening agents on the basis of fatty alcohols and waxy esters.

In view of their application, these lotions are still not fully satisfying in terms of softness and/or sensory impression, processability and stability.

EP A 1 029 977 relates to a composition for treating paper products, such as tissue products, comprising between 30 and 90 % by weight of oil, between 1 and 40 % by weight of wax, between 1 and 30 % by weight of an emulsifying agent and between 5 and 35 % by weight of water. This lotion composition is solid or semisolid at 30°C and remains primarily at the surface of the tissue paper, although it penetrates the tissue paper somewhat more than the solid composition of WO 96/24723 .

DE 199 06 081 A1 discloses emulsions containing (a) 5 to 25 % by weight polyol poly-12-hydroxy stearate, (b) 50 to 90 % by weight waxy esters and (c) 5 to 25 % by weight waxes. This document further contains examples describing the treatment of tissue papers with W/O emulsions as defined above containing about 20 to 25 % water. These compositions are solid or semisolid at 30°C (example 1 corresponds to lotion F of EP A 1 029 977) and show the same penetration behaviour as described above for the lotions of EP A 1 029 977.

WO 02/056842 A2 discloses a W/O emulsion containing
a) polyol poly-12-hydroxy stearate,
b) an oil component selected from glycerides, hydrocarbons, silicon oil, dialkylether and dialkyl carbonates, or mixtures thereof and
c) 5 to 25 weight % water.

It is stated that said emulsion is soft and suitable for use as a body care agent, as well as impregnating and brightening agent for woven fabrics, non-woven fabrics and (tissue) paper.

WO 02/057546 A1 describes a tissue paper penetrated with a lotion composition being a liquid, viscous W/O emulsion comprising
(A) 20 to 75 weight % of at least one oil,
(B) 3 to 40 weight % of at least one non-ionic W/O emulsifier,
(C) optionally 0.5 to 10 weight % of at least one wax,
(D) optionally 1 to 15 weight % of at least one humectant,
(E) 6 to 25 weight % of water.

On a concrete level, it discloses the same lotion composition as WO 02/056842 A2 comprising 20.4 weight % cocoglycerides, 20.4 weight % dicaprylylether, 20.4 weight % polyglycerin-2-dipolyhydroxystearate (Dehymuls® PGPH), 4.8 weight % sorbitan sesquioleate, 3.4 weight % bees wax, 2.0 weight % dicocoyl pentaerythrityl distearyl-citrate, 3.4 weight % aluminium stearate and 7.0 weight % glycerol. Although the lotion described in these WO references shows a good stability under usual conditions, stability problems may occur if it is stored for very long times or at higher temperatures. Further, it was noted that the incorporation of plant extracts into this lotion causes colouring which lowers whiteness (brightness) of tissue paper treated with this lotion. Moreover, improvements in softness also seem to be possible.

In view of the above, it is one object of the present invention to provide a lotioned tissue paper that overcomes disadvantages of prior art formulations.

It is one further object of the present invention to provide a tissue paper treated with a lotion composition wherein that lotion composition shows an excellent stability.

According to one further object, the present invention aims at providing a tissue paper treated with a lotion composition showing an excellent softness (surface and/or bulk softness).

It is one further object of the present invention to provide a lotioned tissue paper showing a high degree of whiteness (brightness).

It is one further object of the present invention to provide a tissue paper treated with a lotion composition that can be applied at relatively low temperatures.

Finally, it is one further object of the present invention to provide a tissue paper treated with a lotion composition wherein said lotion composition shows a suitable, in particular improved balance of critical properties including stability, softness and/or sensory impression, compatibility with plant extracts and application temperature.

### SUMMARY OF THE INVENTION

This technical object is solved by a tissue paper treated with a lotion composition comprising
(A) a hydrocarbon-based oil,
(B) optionally at least one further oil,
(C) 3 to 40 weight % of at least one non-ionic W/O emulsifier,
(D) 0.5 to 10 weight % of at least one coemulsifier based on an ethylene oxide-modified glyceride,
(E) optionally 1 to 15 weight % of at least one humectant,
(F) optionally up to 10 weight % of a metal soap
(G) 6 to 35 weight % of water,
wherein the weight % values relate to the total weight of the lotion composition. This lotion is liquid at room temperature (23°C) and pumpable at 20°C. It preferably consists essentially of the above components and up to 10 weight % other additives, for instance those described below under item 1.8.

It was found that this lotion does not only show an excellent softness and sensory impression, but also a surprisingly good stability, even after storage at room temperature over a longer period of time and/or after medium-long storage at temperatures above room temperature. Another merit of this lotion is excellent compatibility with plant extracts and the resulting high whiteness (brightness) of tissue papers treated therewith.

It is preferred that a tissue paper treated with this lotion, even if containing a plant extract, shows a decrease in whiteness of less than 3.9 %, preferably less than 3.75 %, in particular less than 3.5 %, as measured according to DIN EN 12625-7, item 7.3.2, colour (D65/10°), the whiteness of unlotioned tissue paper being 100 %.

Its fairly low viscosity and good penetration behaviour can be utilized for treating the inner plies of multi-ply tissue papers. Surprisingly, it was found that the lotion used in the present invention displays a viscosity minimum at preferred low application temperatures ranging from 25 to 50°C, in particular 30 to 45°C, e.g. 32 to 40°C, or 32 to 38°C.

Moreover, the lotion is capable of transferring active agents to the skin of the user, if necessary.

### FIGURE

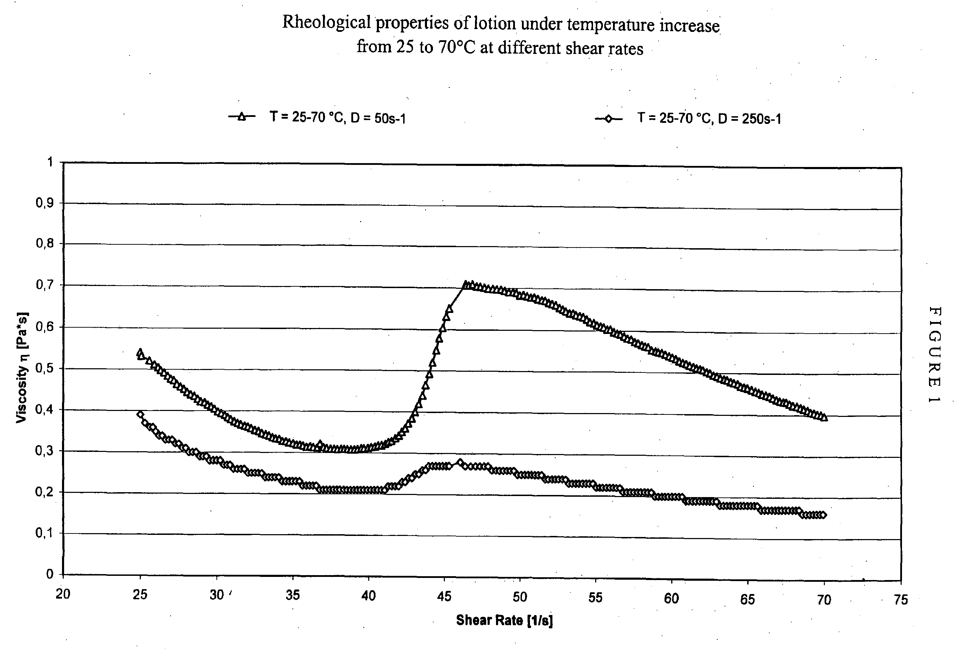

Figure 1 shows the results of two rheology measurements with the lotion of example 1 under different conditions (t denotes the time lapsed during heating from minimum to maximum temperature and D is the shear rate). The upper curve achieved with the lower shear rate of D=50 1/s clearly shows a minimum at the preferred application temperatures.

### DETAILED DESCRIPTION OF THE INVENTION

The lotioned tissue paper of the invention is typically obtained by applying the aforementioned lotion composition to a dry tissue web. Preferably, the residual water content of the tissue web (without lotion) is no more than 10 % by weight.

### 1.Lotion

By mixing and homogenizing the lotion components described in the following, a liquid water-in-oil (W/O) emulsion is obtained which preferably has a viscosity of less than 10,000 mPa•s at 23 ° C, a value typical of semi-solid lotions (measured with a Brookfield-RVF viscosimeter, spindle 5, 10 rpm). Preferably, the viscosity is 500 to 5,000 mPa•s, more preferably 700 to 3000 mPa•s, in particular 1,000 to 2,000 mPa•s, measured at 23° C under the above conditions.

If measured by a rheometer, the lotion shows a viscosity [mP•as] of less than 1, in particular less than 0.8 in the temperature range of 25 to 70°C (shear rate D=50 1/sec, CR mode (rotation), constant heating from 25 to 70°C over 450 sec, number of measuring points 200). The measurement was conducted with a Haake RheoStress RS1 rheometer (now available from Thermo Electron) under the following additional conditions regarding measurement geometry: sensor C35/2° Ti, A-factor of 89090.000 Pa/Nm, M-factor of 28.650 (1/s)/(rad/s), moment of inertia: 1.769e-06 kg m², attenuation of 30.00 and slit width 0.105 mm. The tempering device used was TCP/P (Peltier/Plate).

Despite the low viscosity, the lotion composition is very stable during preparation and use, and does not separate into the water and oil phase, even at temperatures above room temperature and/or after longer storage.

The low viscosity of the lotion is enhanced by the use of a fairly small amount of solid components. Hereinafter, the term "solid" or "liquid" refers to the physical state at room temperature (23° C). Preferably, the overall content of solid components is less than 10 % by weight, in particular, less than 5 % by weight, based on the total weight of the lotion composition.

This lotion does not require the presence of silicone oils or quaternary amine compounds in order to attain its softening effect, although their use is not excluded.

Hereinafter, the term "oil" is used for water-insoluble, organic, natural and synthetic, cosmetically useful oils having preferably a liquid (also viscous) consistency at room temperature (23°C). Moreover, unless stated otherwise, weight % values always relate to the total weight of the lotion composition.

### 1.1. Hydrocarbon-based Oil Component (A)

The total amount of oil components in the lotion composition is preferably 20 to 75 weight % (e.g. 30 to 70 weight %), in particular 45 to 65 weight %.

The lotion comprises as one essential component a hydrocarbon-based oil component (A). The oil component (A) can be properly selected among known cosmetically useful hydrocarbon-based oils, such as cyclic or non-cyclic, saturated or unsaturated aliphatic or aromatic oils.

The hydrocarbon-based oil preferably has from 8 to 32, in particular 15 to 20 carbon atoms. Examples include squalane, squalene, paraffinic oils, isohexadecane, isoeicosane, polydecene or dialkycyclohexane, and mineral oil, mineral oil being preferred .

If the hydrocarbon-based oil component (A) is used as sole oil component, it is preferred to use low viscosity embodiments thereof. According to one preferred embodiment, the hydrocarbon-based oil component (A) is admixed with a second oil component (B) preferably having a lower viscosity than component (A) (measured with a Höppler falling sphere viscosimeter as explained below). In this embodiment, oil component (A) is preferably present in an amount of 20 to 40 weight %, in particular 25 to 35 weight %. The preferred viscosity difference between oils (A) and (B) is at least 5 mPa•s.

Preferably, the oil component (A), or the mixture of oil component (A) and (B) is selected such that the viscosity is 1 to 230 mPa•s, in particular 2 to 200 mPa•s, e.g. 5 to 180 mPa·s or 10 to 150 mPa•s (measured with a Höppler falling sphere viscosimeter at 20°C (method "Deutsche Gesellschaft für Fettchemie" DGF C-IV 7). Preferred mineral oils can be selected from white oil pharma 40 ("Weißöl Pharma 40"), fluid or liquid paraffin oil ("Paraffinöl dünnflüssig"), viscous paraffin oil ("Paraffinöl dickflüssig"), paraffinum liquidum, paraffinum perliquidum or paraffinum subliquidum.

### 1.2. Optional Second Oil Component (B)

The second oil component may be suitably selected from among known oils other than hydrocarbon-based ones, e.g. among oils from plant sources or synthetic oils. If present, oil component (B) is preferably used in amounts of 10 to 40 weight %, in particular 20 to 30 weight %.

If present, the second oil component preferably is selected in a suitable manner among low viscosity oils, i.e oils having a viscosity of 1 to 100 mPa•s, in particular 1 to 50 mPa•s (e.g. 1 to 30 mPa•S) measured with a Höppler falling sphere viscosimeter at 20°C (method "Deutsche Gesellschaft für Fettchemie" DGF C-IV 7), in order to achieve the desired penetration behaviour on tissue.

Preferably, the oil component (B) contains at least one oil selected from among the following types:
- Glycerides, which are mono-, di- and/or tri ester (fatty acid ester) of glycerol (in particular di- and/or triester). Glycerides can be obtained by chemical synthesis or from natural sources (plant or animal) as known in the art. Preferably the fatty acid component has from 6 to 24, more preferably 6 to 18, in particular 8 to 18 carbon atoms. The fatty acid can be branched or unbranched as well as saturated or unsaturated. According to the invention the use of liquid glycerides from plant sources is preferred, in particular the use of a modified liquid coconut oil (INCI name: cocoglycerides, available under the trade name myritol® 331 from Cognis Deutschland GmbH) which contains as main component a mixture of di- and triglycerides based on C8 to C18 fatty acids.
- Natural plant oils which may contain liquid glycerides as main component, such as soja oil, peanut oil, olive oil, sunflower oil, macademia nut oil or jojoba oil.
- Guerbet alcohols; guerbet alcohols are based on fatty alcohols having 6 to 18, preferably 8 to 10 carbon atoms, such as 2-ethylhexanol or 2-octyldodecanol;
- Fatty acid esters, preferably those having 12 to 60 carbon atoms including
   a) esters of linear or branched, saturated or unsaturated C₆-C₂₄ fatty acids and linear or branched, saturated or unsaturated C₆-C₂₄ fatty alcohols (e.g. hexyl laurate, myristyl isostearate, myristyl oleate, cetyl isostearate, cetyl oleate, stearyl isostearate, stearyl oleate, isostearyl myristate, isostearyl palmitate, isostearyl stearate, isostearyliso stearate, isostearyl oleate, oleyl myristate, oleyl isostearate, oleyl oleate, oleyl erucate, erucyl isostearate, erucyl oleate, cococaprylate/caprate).
   b) Esters of C₁₈-C₃₈ alkyl hydroxy carboxylic acids and linear or branched, saturated or unsaturated C₆-C₂₂ fatty alcohols,
   c) esters of linear and/or branched, saturated or unsaturated fatty acids and polyhydric alcohols (such as propylene glycol, dimerdiol or trimertriole) and/or guerbet alcohols, liquid triglycerides or triglyceride mixtures, or liquid mono-/di-triglyceride mixtures.
- Esters from aromatic carboxylic acids, such as esters of C₆-C₂₂ fatty alcohols and/or guerbet alcohols and aromatic carboxylic acids, in particular benzoic acid (e.g. Finsolv ®),
- Esters of dicarboxylic acids, in particular esters of C₂-C₁₂ dicarboxylic acids and linear or branched, saturated or unsaturated alcohols having 1 to 22 carbon atoms or polyols having 2 to 10 carbon atoms and 2 to 6 hydroxy groups.
- Substituted cyclohexanes
- Symmetric or asymmetric, linear or branched dialk(en)ylethers having from 6 to 24 carbon atoms (per alk(en)yl group, preferably having 12 to 24 C atoms as total number of C atoms), such as di-n-octylether (dicaprylylether), di-(2-ethylhexy)ether, laurylmethylether, octylbutylether or didocecylether, the use of di-n-octylether (dicaprylylether; viscosity: 2-5 mPaS at 20°C; DGF method described above) being preferred.
- Dialk(en)ylcarbonates having preferably at least one C6 to 22 alkyl or alkenyl group (preferred total number of C atoms: not more than 45, including the C atom of the carbonate unit). The alkyl or alkenyl group can be straight or branched. The alkenyl unit may display more than one double bond. They can be obtained by transesterification of dimethyl or diethyl carbonate in the presence of C6 to C22 fatty alcohols according to known methods (cf. Chem. Rev. 96, 951 (1996)). Typical examples for dialk(en)ylcarbonates are the (partial) transesterification products of caprone alcohol, capryl alcohol, 2-ethylhexanol, n-decanol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachidyl alcohol, gadoleyl alcohol, behenyl alcohol, erucyl alcohol and brassidyl alcohol as well as their technical mixture, which are for instance obtained by high pressure hydrogenisation of technical methyl ester(s) on fat or oil basis. Particularly suitable in view of their low viscosity at 20°C are dihexyl-, dioctyl-, di-(2-ethylhexyl)- or dioleylcarbonate (viscosity of dioctylcarbonate: 7 mPaS at 20°C; DGF method described above). Thus it is preferred to use either short chain (C6 to C10) alkyl or alkenyl carbonates.
- Ring-opening products of epoxidized fatty acid esters and polyols, and
- silicon oils of linear or cyclic structure such as dimethylpolysiloxane, methylphenylpolysiloxane, cyclomethicone, as well as amino-, fatty acid-, alcohol-, polyether-, epoxy-, fluorine-, glycosyde and/or alkyl-modified silicon oil. Further, dimethicone oils can be used.

Generally, it is preferred to select the liquid embodiments among the above compound types.

Further, the oil component (B) is preferably selected (depending on chain length or esterification degree as known from the prior art) such that its polarity is not greater than 5, in particular not greater than 4 Debey.

Oil component (B) is preferably selected from oils having spreading values of at least 250 mm²/10 min (measured in line with U. Zeidler: Über das Spreiten von Lipiden auf der Haut, Fette-Seifen-Anstrichmittel Nr. 10, 403-408, 1985**).** It is particularly preferred to use ester oils, ether oils, carbonate oils, guerbet alcohols or glycerides having the above spreading value. These include for instance dibutyl adipate, isopropyl palmitate, hexyl laurate, ethylhexyl stearate, dicaprylyl ether, dicaprylyl carbonate, hexyldecyl stearate, oleyl oleate, oleyl erucate or vegetable oils.

The weight proportion of oil component (B) is preferably at least 20 % by weight, in particular at least 40 % by weight based on the total amount of (A) and (B).

### 1.3. W/O emulsifier (C)

The emulsifier or emulsifier composition (C) is of a non-ionic type and has primarily the function of forming a water-in-oil emulsion. It can also contribute to the softness of tissue paper. Preferably, it has a HLB value of 2.5 to 10, in particular 2.5 to 5.

Its content is 3 to 40 % by weight, more preferably 8 to 30 (e.g. 10 to 25), in particular 12 to 21 % by weight.

Preferably a liquid W/O emulsifier is used, although the use of minor amounts of a solid emulsifier is possible, as long as the viscosity of the resulting lotion composition is not too high.

Component (C) can be suitably selected from a liquid polyol polyester wherein a polyol having at least two hydroxy groups is esterified with at least one carboxylic acid having from 6 to 30 carbon atoms. Polyols include monosaccharides, disaccharides, and trisaccharides, sugar alcohols, other sugar derivatives, glycerol, and polyglycerols, e.g. diglycerol, triglycerol, and higher glycerols. Such polyol preferably has from 3 to 12, in particular 3 to 8 hydroxy groups and 2 to 12 carbon atoms (on average, if it is a mixture as in polyglycerols). The polyol preferably is polyglycerol, in particular that having the specific oligomer distribution described in WO 95/34528.

The carboxylic acid used in the polyol polyester preferably is a fatty acid having from 6 to 30 carbon atoms (Hereinafter, unless stated otherwise, the term "fatty acid" is not limited to the naturally occurring, even-numbered, saturated or unsaturated long-chain carboxylic acids, but also includes their uneven-numbered homologues or branched derivatives thereof. It may also include double bonds at positions where they do not naturally occur. Correspondingly, "fatty acid" is used as synonymous of linear or branched, substituted or unsubstituted, saturated or unsaturated aliphatic carboxylic acid. Unless stated otherwise, the fatty acid has from 6 to 30 carbon atoms, preferably from 12 to 24 carbon atoms).

Particularly preferred is a fatty acid containing at least one hydroxy group, a mixture or condensation products (poly(hydroxy fatty acids)) thereof. The preferred carbon range for the above mentioned carboxylic acid, as well as for fatty acids or hydroxy fatty acids, is from 12 to 24, in particular 16 to 18. A particularly preferred poly(hydroxy fatty acid) is the condensation product of hydroxy stearic acid, in particular 12-hydroxy stearic acid, optionally in admixture with poly(ricinoleic acid), said condensation product having the properties described in WO 95/34528**.** Preferred emulsifiers include the polyol poly(hydroxystearates) described in WO 95/34528, in particular polyglycerol poly(hydroxystearates) having the characteristics disclosed in this document, e.g. (polyglycerin-2-dipolyhydroxystearate). This product is commercially available from Cognis Deutschland GmbH under the tradename Dehymuls^{®} PGPH.

The weight ratio of the W/O emulsifier to the oil component(s) preferably ranges from 0.2 to 2.0.

### 1.4. Coemulsifier (D)

The W/O emulsion used in the present invention comprises 0.5 to 10 % by weight, preferably 2 to 8.5 % by weight, in particular 4 to 7 % by weight of at least one coemulsifier based on an ethylene oxide-modified glyceride.

The glyceride component of this coemulsifier is preferably a triglyceride. The fatty acid component of this glyceride preferably has from 12 to 24 carbon atoms. Preferably, the ethylene oxide is chemically linked to the fatty acid portion of the glyceride. This is preferably achieved by using fatty acid components carrying at least one protic functionality, such as hydroxy or amino, hydroxy being preferred. According to preferred embodiments, the fatty acid proportion thus represents a C₁₂-C₂₄, in particular C₁₆-C₂₀ fatty acid which may be unsaturated and carries at least one hydroxy group on the fatty acid chain. One preferred example for a hydroxy fatty acid of this type is (R)-(Z)-12 hydroxy-9-octadecenoic acid (ricinoleic acid) and more preferably the hydrogenated (saturated) derivative thereof. Moreover, it .is preferred that one mol of glyceride is modified with 2 to 15 mol (in average) ethylene oxide, in particular 5 to 9 mol ethylene oxide.

It should be noted that, as coemulsifier (D), commercially available mixtures can be used which contain the ethylene oxide-modified glyceride as a major component (more than 50 % by weight, in particular more than 75 % by weight). One example for commercially available products of this type is Dehymuls^{®} HRE-7 (available from Cognis Deutschland GmbH).

### 1.5. Optional Humectant (E)

The lotion composition may comprise from 1 to 15% by weight, preferably 2 to 7% by weight and in particular 3 to 5 % by weight (water-soluble) humectant (e.g. 3.5 to 4.5 % by weight).

The humectant performs multiple functions. First, it binds water and counteracts the tendency of water to evaporate. Moreover, the humectant may interact with other lotion components and can contribute to the softness of the tissue paper, in particular its bulk softness.

The humectant preferably is a polyhydroxy compound, which is understood to be an organic compound having at least two hydroxy groups and which preferably consists only of carbon, hydrogen, oxygen and nitrogen, in particular only of C, H and O. It is further desirable that the humectant is not ionic.

Although hydrophilic surfactants (having a HLB number of 10 or greater, see for instance US 4,764,418) can have humectant properties, it is preferred according to the invention that the humectant be free of major hydrophobic molecule parts, e.g. fatty acid or fatty alcohol residues.

Further, the humectant preferably has a liquid consistency, even though it is possible to use a minor amount of a solid, low melting point humectant if the weight proportion of the remaining solid lotion ingredients is low.

If liquid humectants are to be employed, the molecular weight (weight average) preferably is less than 1,000, more preferably less than 800, and in particular not more than 600.

Examples of suitable humectants include: glycerol, diglycerol, triglycerol, ethylene glycol, propylene glycol, butylene glycol, 1,2,6 hexanetriol, polyalkylene glycols, e.g. polyethylene glycol or polypropylene glycol, for instance polyethylene glycol having a weight average molecular weight of from about 200 to 600; neopentyl alcohols such as pentaerythritol or neopentyl glycol; sugar alcohols such as threitol, erythritol, adonitol (ribitol), arabitol, xylitol, dulcitol, mannitol and sorbitol, carbohydrates such as D (+)- glucose, D (+)- fructose, D (+)- galactose, D (+)- mannose, L-gulose, saccharose, galactose, maltose, polyglycerols, polyoxypropylene adducts of glycerol, methoxypolyethylene glycol, polyethylene glycol ethers of sugar alcohols, such as sorbitol, polyethylene glycol ethers of glycerol, and combinations thereof. Hyaluronic acid may also be used as humectant.

One preferred humectant is glycerol.

### 1.6. Metal soaps (F) (optional)

In order to stabilize the W/O emulsion, further a metal soap of the following preferred formula may be used (up to 10 % by weight), preferably in an amount of 0.5 to 5, in particular 3 to 4 % by weight:

(R¹COO)ₙ-X

wherein R¹CO represents a fatty acid-derived acyl group, in particular a linear, saturated or unsaturated acyl residue having 6 to 24 carbon atoms and optionally a hydroxy group, preferably one having 12 to 20 carbon atoms, X is an alkali metal (e.g. Li), an earth alkali metal (e.g. Ca, Mg), Al or Zn and n is the valence of X. Preferred examples of the metal soap involve zinc, calcium, magnesium or aluminium stearate.

### 1.7. Water (G)

The lotion composition contains 6 to 35 % by weight, more preferably 10 to 30% by weight, in particular 15 to 25 % by weight of water. The water contributes to a lotion-like pleasant feel on the skin of the user, for instance during blowing the nose with a treated handkerchief. Water counteracts further the tendency of pure humectants to withdraw water from the human skin. On the other hand, the water content should not be much higher than 35 % by weight, since then the mechanical strength of the treated tissue paper may suffer.

It is possible to determine the water content in the lotion composition by conducting a water determination according to Karl Fischer. This may also be done with the treated tissue paper. Then the entire lotion is extracted with suitable organic solvents, such as water-free ethanol followed by the water determination of the ethanol extract according to Karl Fischer. If necessary, the residual water content of the treated tissue paper is to be subtracted.

### 1.8. Optional Additives (H)

Optionally, the lotion composition may contain up to 10 % by weight, in particular 0.01 to 5 % by weight additives (e.g. 0,05 to 3 % by weight), such as
- Preservatives which stabilize the lotion composition, such as methylisothiazolin(on) which may have a chlorine as substituent, e.g. 5-chloro-2-methyl-4-isothiazolin-3-on or 2-methyl-4-isothiazolin-3-on; phenoxyethanol or PHB ester, paraben preservatives, pentanediol, sorbic acid or other compounds as mentioned in "*Kosmetikverordnung* (Cosmetics Regulation), Anlage 4, Teil A und B".
- Germicidal agent(s), e.g. those described in DE-199 06 081 A.
- Cosmetic agents, preferably from natural sources (plant extracts), having for instance a skin-soothing, antiphlogistic (reduction of skin irritation), wound-healing, cell-regenerating, anti-inflammatory and/or anti-itch effect such as allantoin; aloe vera extract; chamomile extract containing azulene and α-bisabolol; echinacea; dragosantol; panthenol; liquorice root extract containing 18-glycyrrhetinic acid; lime tree extract containing quercetin and/or glyco-rutin; marigold (calendula oil); lime-blossom extract; sage extract, melissa extract; urea; phytosterols, optionally ethoxylated (available from Henkel under the tradename "Generol"); chitosan (acetylated chitin); anthocyanidins; ginkgo leaf extract containing quercetin and rutin; horse chestnut containing quercetin and campherol; vitamins or provitamins such as provitamin B5 or Vitamin E; avocado oil; birch extract; arnica; extract of rose of Sharon or St. John's wort; teatree oil; cucumber, hops, or hamamelis extracts or ingredients, ethoxylated quaternary amines; wound-healing protein hydrolysates;
- Perfume, e.g. those described in DE 199 06 081; and/or
- Cosmetically useful dyes and pigments, e.g. those described in ***"***Kosmetische Färbemittel" (Cosmetic coloring agents), Verlag Chemie, Weinheim, 1984, p. 81-106" )", published by the "Farbstoffkommission der Deutschen Farbstoffgemeinschaft**".**

One preferred type of additives are plant extracts of the type as already mentioned above which often contain one or more wound-healing/soothing agents. Typically these extracts are prepared by extracting the entire plant. In some cases it can also be preferred to use solely the blossom and/or leaves of the plant. Preferred extracts are obtained from chamomile aloe vera, hamamelis, lime-blossom, sage and melissa. It is one additional merit of the present invention that plant extracts can be used without undesired coloring reactions of the lotion.

The above additives may be used separately or in combination.

### 1.9. Most preferred lotion

The most preferred lotion composition, which based on current knowledge reflects the best mode for carrying out the invention, comprises the following components:
(A) 25 to 35 weight % mineral oil,
(B) 20 to 30 weight % of a further oil component selected from liquid synthetic triglyceride mixtures; vegetable oils; guerbet alcohols; liquid esters; liquid substituted cyclohexanes; symmetric or asymmetric dialk(en)ylethers having from 6 to 22 C atoms per alk(en)yl group; linear or branched dialk(en)ylcarbonate derived from C6 to C22 fatty alcohols; ring-opening products of epoxidized fatty acid esters and polyols; and silicone oils;
(C) 12 to 21 weight % of a W/O emulsifier selected from liquid polyol polyester wherein a polyhydric alcohol having at least two hydroxy groups is esterified with at least one acid having from 6 to 30 carbon atoms,
(D) 4 to 7 weight % coemulsifier based on a glyceride comprising a hydroxy fatty acid being modified with the adduct of 2 to 15 mol ethylene oxide,
(E) optionally 1 to 10 weight % humectant,
(F) optionally 0.5 to 5 weight % of a metal soap
(G) 10 to 30 weight % water,
(H) optionally 0.1 to 5 weight % additives.

### 2. Preparation of lotion

The lotion composition (water-in-oil emulsion) can be prepared according to known methods.

One procedure involves mixing and homogeneously stirring the oil phase components, such as the oil components (A) and (B), and the emulsifiers (C) and (D) and other optional oil-soluble additives at room temperature (usually for approximately 10 min). These components are typically highly soluble and give rise to a homogeneous mixture. The components of the water phase such as water, humectant, and possible water-soluble or water-dispersible additives such as perfume or preservatives are separately mixed at room temperature and slowly added to the mixture of oil-phase components during continuous stirring. After continued stirring (preferably for approximately 10 min) the resulting mixture is then homogenized (usually for approximately 10 min) with a suitable dispersion device such as supraton or stator-rotor homogenizers of Ultraturrax type (see for instance Karlheinz Schrader, Grundlagen und Rezepturen der Kosmetika, Hüthig Buch Verlag Heidelberg, Second Edition, 1989, pages 906 to 912**).** As known from the prior art, homogenizing conditions may have an impact on the viscosity of the emulsion obtained. If the viscosity is too high, which is undesirable in the present invention, it is possible for instance to reduce the energy influx during homogenization, in particular by lowering the rotational speed of the rotor/stator system.

Further, it is possible to prepare the lotion composition of the invention by mixing the oil phase and water phase components at a higher temperature. For this purpose, it is preferred to heat the oil phase and water phase components separately to about 80°C to 85°C. Then, at this temperature the water phase components are slowly added to the oil phase components while stirring, optionally homogenizing. After continued stirring, preferably for about 5 min, the mixture is allowed to cool while stirring in such a way that it remains in continuous motion. Simultaneously, the incorporation of air should be avoided. The mixture is then homogenized with a suitable dispersion device such as supraton or stator-rotor homogenizers of Ultraturrax type, preferably at 60° to 65° C, in order to improve stability and structure. After a homogeneous state is reached, the composition is allowed to cool to room temperature.

### 3. Tissue paper to be treated

Based on the underlying compatibility of the production processes (wet laying), "tissue" production is counted among the paper making techniques. The production of tissue is distinguished from paper production by its extremely low basis weight of normally 80 g/m² or less, in particular 65 g/m² or less and its much higher tensile energy absorption index. The tensile energy absorption index is arrived at from the tensile energy absorption in which the tensile energy absorption is related to the test sample volume before inspection (length, width, thickness of sample between the clamps before tensile load). Paper and tissue paper also differ in general with regard to the modulus of elasticity that characterizes the stress-strain properties of these planar products as a material parameter.

A tissue's high tensile energy absorption index results from the outer or inner creping. The former is produced by compression of the paper web adhering to a dry cylinder as a result of the action of a crepe doctor or in the latter instance as a result of a difference in speed between two wires ("fabrics"). In the latter technique, often referred to as "(wet) rush transfer", for instance the forming fabric of the paper machine is moved at greater speed than that of the fabric to which the formed paper web is transferred, for instance a transfer fabric or a TAD fabric (through air drying), so that the paper web is somewhat bundled when it is taken up by the transfer fabric. Many prior art documents (e.g. EP-A-0 617 164**,** WO-94/28244**,** US-5 607 551**,** EP-A-0 677 612**,** WO-96/09435) refer to this as "inner creping", when they describe the production of "uncreped" tissue paper by rush transfer techniques. The inner and outer creping causes the still moist, plastically deformable paper web to be internally broken up by compression and shearing, thereby rendering it more stretchable under load than uncreped paper. Most of the functional properties typical of tissue and tissue products result from a high tensile energy absorption index (see DIN EN 12625-4 and DIN EN 12625-5).

Typical properties of tissue paper include the ready ability to absorb tensile stress energy, their drapability, good textile-like flexibility, a high specific volume with a perceptible thickness, as high a liquid absorbency as possible and, depending on the application, a suitable wet and dry strength as well as an interesting visual appearance of the outer product surface.

According to the invention creped or "uncreped" tissue paper can be lotioned, the use of creped tissue paper being preferred. The tissue paper (or the final tissue paper product obtained therefrom) can be single-ply or multi-ply (typically 2 to 6). The penetration behavior of the lotion is particular suitable for multi-ply tissues (or tissue products), in particular 4-ply embodiments as used in handkerchiefs, since the lotion can be almost evenly distributed over the outer and inner plies. The tissue paper may be homogeneous or layered, wet-pressed or blow-dried (TAD-dried). The tissue paper includes, but is not limited to, felt-pressed tissue paper, pattern-densified tissue paper, uncompacted tissue paper or compacted tissue paper.

The starting material for the production of the tissue paper usually is a fibrous cellulosic material, in particular pulp.
If, however, linters or cotton is used as raw material for the production of tissue paper, usually no further pulping steps are needed. Due to the morphological structure, the cellulose already exists in an open state.

The starting pulps used may relate to primary fibrous materials (raw pulps) or to secondary fibrous materials, whereby a secondary fibrous material is defined as a fibrous raw material recovered from a recycling process. The primary fibrous materials may relate both to a chemically digested pulp and to mechanical pulp such as thermorefiner mechanical pulp (TMP), chemothermorefiner mechanical pulp (CTMP) or high temperature chemithermomechanical pulp (HTCTMP). Synthetic cellulose-containing fibres can also be used. Preference is nevertheless given to the use of pulp from plant material, particularly wood-forming plants. Fibers of softwood (usually originating from conifers), hardwood (usually originating from deciduous trees) or from cotton linters can be used for example. Fibres from esparto (alfa) grass, bagasse (cereal straw, rice straw, bamboo, hemp), kemp fibers, flax, and other woody and cellulosic fiber sources can also be used as raw materials. The corresponding fiber source is chosen in accordance with the desired properties of the end product in a manner known from the prior art. For example, the fibers present in hardwood, which are shorter than those of softwood, lend the final product a higher stability on account of the higher diameter/length ratio. If softness of the product is to be promoted, which is important *e.g.* for tissue paper, eucalyptus wood is particularly suitable as a fiber source.

With regard to softness of the products, the use of chemical raw pulps is also preferred, whereby it is possible to use completely bleached, partially bleached, and unbleached fibers. The chemical raw pulps suitable according to the invention include *inter alia,* sulphite pulps, kraft pulps (sulphate process).

Before a raw pulp is used in the tissue making process, it may also be advantageous to allow further delignification to occur in a separate process step or employ a bleaching process to achieve a more extensive removal of lignin after the cooking process and to obtain a completely cooked pulp.

A preferred production process for tissue paper uses
a a forming section (for wet-laying a slurry of cellulosic fibrous material, typically pulp) comprising a headbox and wire portion, and
b the drying section (TAD (through air drying) or conventional drying on the yankee cylinder) that also usually includes the crepe process essential for tissues.

This is typically followed by
c the monitoring and winding area.

The tissue paper can be formed by placing the fibers, in an oriented or random manner, on one or between two continuously revolving wires of a paper-making machine while simultaneously removing the main quantity of water of dilution until dry-solid contents of usually between 12 and 35 % are obtained. It is possible to include additives in the paper furnish to improve the wet-strength or dry-strength or other properties of the finished tissue paper.

Drying the formed primary fibrous web occurs in one or more steps by mechanical and thermal means until a final dry- solids content of usually about 93 to 97 % is obtained. In the case of tissue making, this stage is followed by the crepe process which crucially influences the properties of the finished tissue product in conventional processes. The conventional dry crepe process involves creping on a drying cylinder having a diameter of usually 4.5 to 6 m , the so-called yankee cylinder, by means of a crepe doctor with the aforementioned final dry-solids content of the base ("raw tissue") tissue paper (wet creping can be used if lower demands are made of the tissue quality). The creped, finally dry base tissue paper is then available for further processing into the paper product or tissue paper product according to the invention.

Instead of the conventional tissue making process described above, the invention gives preference to the use of a modified technique in which an improvement in specific volume is achieved by a special kind of drying within process section b and in this way an improvement in bulk softness of the resulting tissue paper is achieved. This pre-drying process, which exists in a variety of subtypes, is termed the TAD (through air drying) technique. It is characterized by the fact that the "primary" fibrous web (like a non-woven) that leaves the sheet making stage is pre-dried to a dry-solids content of about 80 % before final contact drying on the yankee cylinder by blowing hot air through the fibrous web. The fibrous web is supported by an air-permeable wire or belt and during its transport is guided over the surface of an air-permeable rotating cylinder drum. Structuring the supporting imprinting fabric or belt makes it possible to produce any pattern of compressed and uncompressed zones achieved by deflection of the fibres in the moist state, followed by pre-drying (TAD step) and leading the web through a pressure nip between a pressure roll and the Yankee cylinder surface, thereby resulting in increased mean specific volumes and consequently leading to an increase in bulk softness without decisively decreasing the strength of the fibrous web.

Another possible influence on softness and strength of base tissue lies in the production of a layering in which the primary fibrous web to be formed is built up by a specially constructed headbox in the form of physically different layers of fibrous material, these layers being jointly supplied as a pulp jet to the forming stage.

The one-ply intermediate products originating from the paper-making machine and made of lightweight paper usually dry-creped on a yankee cylinder by means of a crepe doctor are generally described as "tissue paper" or more accurately base tissue paper. The one-ply base tissue may be built up of one or a plurality of layers respectively.

All one-ply or multi-ply final products made of base tissue and tailored to the end user's needs, i.e. manufactured with a wide variety of requirements in mind, are known as "tissue products".

When processing the fibrous web or base tissue paper into the final tissue product, the following procedural steps are normally used individually or in combination: cutting to size (longitudinally and/or cross cutting), producing a plurality of plies, producing mechanical and/or chemical ply adhesion, volumetric and structural embossing, folding, imprinting, perforating, application of lotions, smoothing, stacking, rolling up.

To produce multi-ply tissue paper products, such as handkerchiefs, toilet paper, towels or kitchen towels, an intermediate step preferably occurs with so-called doubling in which the base tissue in the finished product's desired number of plies is usually gathered on a common multiply master roll.

The processing step from the base tissue that has already been optionally wound up in several plies to the finished tissue product occurs in processing machines which include operations such as repeated smoothing of the tissue, edge embossing, to an extent combined with full area and/or local application of adhesive to produce ply adhesion of the individual plies (base tissue) to be combined together, as well as longitudinal cut, folding, cross cut, placement and bringing together a plurality of individual tissues and their packaging as well as bringing them together to form larger surrounding packaging or bundles. The individual paper ply webs can also be pre-embossed and then combined in a roll gap according to the foot-to-foot or nested methods.

Embossing can be used for generating ply adhesion in multi-ply tissue papers. In order to ensure that the lotion does not lower the ply adhesion, the embossed regions may be left untreated. Further it is known from US-4,867,831 to use melted thermoplastics to achieve plybonding in lotioned tissue papers.

Tissue products using the lotioned tissue of the invention are preferably sanitary products (e.g. toilet paper), paper handkerchiefs, cosmetic wipes (facials) or as serviettes/napkins. The use in handkerchiefs is preferred.

According to the invention the tissue paper to be treated with the lotion preferably has a basis weight of 8 to 40 g/m², more preferably 10 to 30 g/m², even more preferably 12 to 20 g/m² per ply, in particular 13 to 17 g/m² and a total basis weight (including all plies without lotion) of usually 10 to 80 g/m².

### 4. Application of Lotion on the Tissue Paper

As mentioned, lotion application typically takes place after the paper web has been dried. A suitable point in time is for example directly after drying the web, shortly before combining the webs to form multiple plies or before forming the multi-ply web into the final tissue product. However, it is preferred first to laminate at least two single ply webs to a multiply web, and then to apply the lotion. For tissue paper having two or more plies, the composition may be applied to each ply or only to one or both outer plies. In a preferred production process for lotioned 4-ply tissue (products), two 2-ply webs are each lotioned on only one side, followed by joining together the untreated sides of said 2-ply webs, thereby obtaining a 4 ply product. Generally, it is preferred for the lotion composition to be applied to at least one, preferably both outer plies of multi-ply tissue webs, since then the advantageous penetration behaviour of the lotion composition can fully be developed by achieving as even a distribution as possible with respect to the z-direction (perpendicular) of the multi-ply tissue paper. The individual plies or the multi-ply structure may be patterned either before or after application of the lotion composition. Suitable application techniques include spraying, rotogravure printing or flexographic printing or application by means of rolls having a smooth surface. Preferably, the lotion composition is slightly heated, in particular to a temperature from 25° to 50° C, preferably between 30 and 45°C before it is applied to the paper web.

Preferably, the lotion is applied in an amount of 3 to 10 g per m² treated surface, *i.e*. with double the amount if both surfaces are lotioned being preferred. The weight ratio lotion composition/tissue (single or multi-ply) is preferably 10 to 40 %, more preferably 21 to 35 %, in particular 25 to 30 %. With four-ply products a particularly good and even distribution of the lotion composition is observed when the weight ratio is from 22 to 30 %, in particular 27 to 29 %.

### 5. Example

The following two lotions were prepared at room temperature by mixing and homogenizing the respective components. The lotion referred to as comparative example 1 corresponds to a lotion described in WO 02/056842 A2 and WO 02/057546 A1. Both lotions were assessed with respect to their stability, whiteness (according to DIN EN 12625-7, item 7.3.2, colour (D65/10°)) and sensory impression. As a reference point for the whiteness analysis, the whiteness of unlotioned tissue paper was taken as 100%. In accordance with the present invention, it is preferred that the decrease in whiteness is less than 3.9%, preferably less than 3.75, in particular less than 3.5%. The inventive example 1 satisfied this requirement.

For the stability tests samples of the produced lotions were stored under the conditions given in Table 1. Where phase separation was observed with the naked eye, the lotion was rated " - ".

The sensory test was conducted by an experienced panel of test persons. The optimum sensory impression was rated "1" whilst a poor sensory impression is reflected by "6".

**Table 1**

| **Ingredients** | **Example 1** | **Comparative Example 1** |
|---|---|---|
| Polyglycerin-2-dipolyhydroxystearate | 15 | 20,6 |
| Hydrogenated castor oil + 7EO | 5 | - |
| Mineral oil | 30 | - |
| Ethylhexyl stearate | 22,4 | - |
| Cocoglyceride | - | 20,6 |
| Dicaprylyl ether | - | 20,6 |
| Sorbitan sesquiolate | - | 4,8 |
| Beeswax | - | 3,35 |
| Dicocoyl pentaerythrityl distearyl citrate | - | 2 |
| Tocopheryl and hydrogenated palm glyceride citrate | - | 0,02 |
| Glycerol | 4 | 7 |
| Magnesium stearate | 3,5 | - |
| Aluminium stearate | - | 2,75 |
| Blossom extract from chamomilla recutita (matricaria) | 0,1 | 0,1 |
| Water | 20 | 18,2 |
| Preservative | As required | |
| | | |
| Appearance | milky-white | milky-yellow |
| | | |
| Stability 4 weeks-RT | + | + |
| Stability 8 weeks-RT | + | - |
| Stability 4 weeks-30°C | + | - |
| | | |
| Decrease in whiteness (%) | 3,4 | 3,9 |
| | | |
| Sensory impression of lotion-creaminess | 1,0 | 4,0 |

## Claims

1. Tissue paper comprising a lotion composition, said lotion composition being a W/O emulsion comprising
(A) a hydrocarbon-based oil,
(B) optionally at least one further oil,
(C) 3 to 40 weight % of at least one non-ionic W/O emulsifier,
(D) 0.5 to 10 weight % of at least one coemulsifier based on an ethylene oxide-modified glyceride,
(E) optionally 1 to 15 weight % of at least one humectant,
(F) optionally up to 10 weight % of a metal soap
(G) 6 to 35 weight % of water,
wherein the weight % values relate to the total weight of the lotion composition.

2. Tissue paper according to claim 1, wherein the oil component (A) is mineral oil.

3. Tissue paper according to claim 1, wherein said lotion composition comprises at least one oil (B) having a viscosity of 1 to 100 mPa·s measured with a Höppler falling sphere viscosimeter at 20°C (method DGF C-IV 7).

4. Tissue paper according to claim 1 or 3, wherein said oil component (B) is selected from liquid synthetic triglyceride mixtures; vegetable oils; guerbet alcohols; liquid esters; liquid substituted cyclohexanes; symmetric or asymmetric dialk(en)ylethers having from 6 to 22 C atoms per alk(en)yl group; linear or branched dialk(en)ylcarbonates derived from C 6 to 22 fatty alcohols; ring-opening products of epoxidized fatty acid esters and polyols; and silicone oils.

5. Tissue paper according to claim 1, wherein the non-ionic surfactant (C) comprises a liquid polyol polyester wherein a polyhydric alcohol having at least two hydroxy groups is esterified with at least one acid having from 6 to 30 carbon atoms.

6. Tissue paper according to Claim 5, wherein said polyol polyester is polyglycerin-2-dipolyhydroxystearate.

7. Tissue paper according to claim 1, wherein the glyceride component of coemulsifier (D) comprises a hydroxy-fatty acid.

8. Tissue paper according to claim 1 or 7, wherein the ethylene oxide-modified glyceride is obtainable by reacting 1 mol glyceride with in average 2 to 15 mol ethylene oxide.

9. Tissue paper according to any of claims 1, 7 or 8, wherein the coemulsifier (D) is ethylene oxide-modified hydrogenated castor oil.

10. Tissue paper according to any of claims 1 through 9, wherein the humectant is glycerol.

11. Tissue paper according to claim 1, wherein the lotion composition comprises:
(A) 25 to 35 weight % mineral oil,
(B) 20 to 30 weight % of a further oil component selected from liquid synthetic triglyceride mixtures; vegetable oils; guerbet alcohols; liquid esters; liquid substituted cyclohexanes; symmetric or asymmetric dialk(en)ylethers having from 6 to 22 C atoms per alk(en)yl group; linear or branched dialk(en)ylcarbonate derived from C 6 to 22 fatty alcohols; ring-opening products of epoxidized fatty acid esters and polyols; and silicone oils;
(C) 12 to 21 weight % of a W/O emulsifier selected from liquid polyol polyester wherein a polyhydric alcohol having at least two hydroxy groups is esterified with at least one acid having from 6 to 30 carbon atoms,
(D) 4 to 7 weight % coemulsifier based on a glyceride comprising a hydroxy fatty acid being modified with the adduct of 2 to 15 mol ethylene oxide,
(E) optionally 1 to 10 weight % humectant,
(F) optionally 0.5 to 5 weight % of a metal soap
(G) 10 to 30 weight % water,
(H) optionally 0.1 to 5 weight % additives.

12. Process for preparing a tissue paper according to any of claims 1 to 11, wherein a lotion composition as defined in any of claims 1 to 11 is applied to a tissue paper web.

13. Process according to claim 12, wherein the lotion composition is applied at a temperature from 25 to 50°C to the tissue paper web.
